(19) Europäisches Patentamt
European Patent Office
Office européen des brevets

(11) EP 1 535 657 B1

(12) EUROPEAN PATENT SPECIFICATION

(45) Date of publication and mention of the grant of the patent:
**26.09.2018 Bulletin 2018/39**

(21) Application number: **03792729.0**

(22) Date of filing: **20.08.2003**

(51) Int Cl.:
***B01D 67/00*** *(2006.01)* ***B01D 63/02*** *(2006.01)*
***B01D 65/02*** *(2006.01)* ***B01D 69/08*** *(2006.01)*
***B01D 71/40*** *(2006.01)* ***C08J 7/18*** *(2006.01)*
***C08J 7/00*** *(2006.01)* ***A61L 33/00*** *(2006.01)*
***B01D 71/68*** *(2006.01)*

(86) International application number:
**PCT/JP2003/010488**

(87) International publication number:
**WO 2004/018085 (04.03.2004 Gazette 2004/10)**

(54) **PROCESS FOR PRODUCING MODIFIED SUBSTRATE**

VERFAHREN ZUR ERZEUGUNG EINES MODIFIZIERTEN SUBSTRATS

PROCÉDÉ DE PRODUCTION D'UN SUBSTRAT MODIFIÉ

(84) Designated Contracting States:
**AT BE BG CH CY CZ DE DK EE ES FI FR GB GR HU IE IT LI LU MC NL PT RO SE SI SK TR**

(30) Priority: **21.08.2002 JP 2002240247**

(43) Date of publication of application:
**01.06.2005 Bulletin 2005/22**

(60) Divisional application:
**12183460.0 / 2 543 428**

(73) Proprietor: **TORAY INDUSTRIES, INC.**
**Tokyo 103-8666 (JP)**

(72) Inventors:
- **UENO, Yoshiyuki**
  **Otsu-shi, Shiga 520-0842 (JP)**
- **TAKAHASHI, Hiroshi**
  **Kyoto-shi, Kyoto 607-8088 (JP)**
- **SUGAYA, Hiroyuki**
  **Otsu-shi, Shiga 520-2101 (JP)**

(74) Representative: **Webster, Jeremy Mark et al**
**Mewburn Ellis LLP**
**City Tower**
**40 Basinghall Street**
**London EC2V 5DE (GB)**

(56) References cited:

| | |
|---|---|
| EP-A2- 0 472 936 | WO-A2-02/062880 |
| JP-A- 6 238 139 | JP-A- 11 047 570 |
| JP-A- 53 134 876 | JP-A- 63 097 634 |
| JP-A- 63 097 634 | JP-A- 63 209 663 |
| JP-A- 2000 237 557 | JP-A- 2000 237 557 |
| JP-B2- 3 076 080 | US-A- 5 240 747 |
| US-A- 5 658 466 | US-A- 5 658 466 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 535 657 B1

## Description

Technical Field

The present invention relates to a method for producing modified substrate wherein the surface thereof is subjected to a hydrophilization treatment. The modified substrate can be preferably used in medical devices. Preferably, the modified substrate can also be used as, for example, separation membranes for water treatment, separation membranes of biogenic substances, instruments used for biological experiments, bioreactors, molecular motors, drug delivery systems (DDS), protein chips, DNA chips, biosensors, or components of analytical instruments. In particular, the modified substrate produced by the method of the present invention is preferably used for applications in which the substrate is brought into contact with a biogenic substance, for example, a module for blood purification such as an artificial kidney.

Background Art

In medical devices that are in contact with a body fluid, for example, an artificial blood vessel, a catheter, a blood bag, a contact lens, an intraocular lens, and an artificial kidney, biocompatibility, in particular, hematologic compatibility is an important problem. For example, in separation membranes used for blood purification, adhesion of proteins, or adhesion or activation of blood platelets causes blood clotting. It is known that performing a hydrophilization treatment on the surface of a substrate is effective in remedying such a problem of hematologic compatibility. For example, polysulfone polymers are used as a material for the separation membranes for blood purification. In order to provide a polysulfone with hematologic compatibility, a hydrophilic polymer such as polyvinylpyrrolidone is mixed in the stock solution for preparation of the membrane. Although this method provides hematologic compatibility to some degree, the hematologic compatibility is not sufficient.

In a method disclosed in Japanese Unexamined Patent Application Publication No. 10-118472, in order to improve hematologic compatibility on the surface of a substrate, a polysulfone separation membrane is brought into contact with a solution of a hydrophilic polymer such as polyvinylpyrrolidone. Thus, the separation membrane physically adsorbs the hydrophilic polymer. However, in this method, the hydrophilic polymer is only adsorbed on the surface. Therefore, when the separation membrane is in contact with blood, the hydrophilic polymer may be dissolved into the blood. In a method disclosed in Japanese Unexamined Patent Application Publication No. 6-238139, a polysulfone separation membrane is brought into contact with a solution of a hydrophilic polymer such as polyvinylpyrrolidone. In this method, an insolubilized hydrophilic polymer layer is formed on the surface of the membrane utilizing radiation crosslinking. This method suppresses the dissolution of the hydrophilic polymer. However, when the membrane is in contact with blood, the insolubilized hydrophilic polymer activates the blood platelets. As a result, hematologic compatibility is deteriorated rather than improved.

Processes for modifying surfaces of materials involving irradiation with ultraviolet rays are disclosed in US-A-5240747, EP-A-0472936 and WO02/062880.

JP-A-63097634, US-A-5658466 and JP-A-2000237557 disclose processes in which membranes are irradiated with $\gamma$-rays.

It is an object of the present invention to provide a modified substrate having high hematologic compatibility wherein a hydrophilic polymer is immobilized on the surface of the substrate, and a method for producing the same. As a result of intensive study, the present inventors have found a method for immobilizing a hydrophilic polymer on a substrate without excessive crosslinking or degrading the hydrophilic polymer, and have accomplished the present invention.

In a first aspect the invention provides a method for producing a modified substrate as defined in the accompanying claim 1.

Thus, the present invention provides a method for producing a modified substrate comprising a step of irradiating the substrate with $\gamma$-ray radiation with an absorbed dose of at least 20 kGy while the substrate is brought into contact with an aqueous solution containing a hydrophilic polymer and an antioxidant, wherein the hydrophilic polymer is a polyvinylpyrrolidone or a polyvinylpyrrolidone copolymer, and the substrate is a separation membrane system comprising polysulfone and polyvinylpyrrolidone,
wherein the antioxidant is selected from alcohols and inorganic salts, wherein the alcohols are selected from methanol, ethanol, propanol, ethylene glycol and glycerine, and the inorganic salts are selected from sodium hydrosulfite, sodium pyrosulfite, and sodium dithionate,
and wherein the obtained modified substrate has a soluble hydrophilic polymer ratio of 15 weight percent or less, a number of adhered human blood platelets adhered in an area of $4.3\times10^3$ $\mu m^2$ of 10 or less, and a surface hydrophilic polymer ratio measured by X-ray photoelectron spectrometry of at least 20 weight percent, wherein the soluble hydrophilic polymer ratio is defined as the ratio of soluble hydrophilic polymer which is soluble in a solvent that dissolves the hydrophilic polymer but does not dissolve the substrate to the total of the hydrophilic polymer in the modified substrate,

and wherein the number of adhered blood platelets is defined as the number of blood platelets adhered on the surface of the modified substrate when it is brought into contact with blood for one hour.

Brief Description of the Drawing

Fig. 1 is a view showing an example of the basic structure of an artificial kidney system.

Best Mode for Carrying Out the Invention

In the present invention, a substrate is irradiated with radiation while the substrate is brought into contact with an aqueous solution of a hydrophilic polymer, thus producing a modified substrate wherein the hydrophilic polymer is immobilized on the surface of the substrate. Hematologic compatibility of a substrate depends on the surface state of areas that are in contact with blood. In general, the higher the hydrophilicity of the surface and the higher the mobility of the hydrophilic polymer immobilized on the surface, the higher the hematologic compatibility of the substrate is. This is because a hydrophilic polymer having high mobility eliminates proteins or blood platelets due to its molecular motion.

Herein, the term immobilization refers to a state in which a hydrophilic polymer is bonded with a substrate. In the present invention, it is necessary for the soluble hydrophilic polymer ratio to be 15 weight percent or less, and preferably, 10 weight percent or less. Herein, the term soluble hydrophilic polymer refers to a hydrophilic polymer that is neither crosslinked nor insolubilized due to immobilization on the substrate. The soluble hydrophilic polymer ratio is defined as a ratio of the soluble hydrophilic polymer to the total of the hydrophilic polymer in the modified substrate. A detailed method for measuring the soluble hydrophilic polymer ratio will be described later. When the soluble hydrophilic polymer ratio exceeds 15 weight percent, bonding of the hydrophilic polymer with the substrate is insufficient. Therefore, when the modified substrate is brought into contact with blood, the hydrophilic polymer may be dissolved into the blood.

The amount of dissolution of the hydrophilic polymer is preferably 0.5 $mg/m^2$ or less, more preferably, 0.3 $mg/m^2$ or less. Herein, the amount of dissolution of the hydrophilic polymer is defined as follows: A substrate is brought into contact with purified water at 37°C for 4 hours. The amount of the hydrophilic polymer that is dissolved into the purified water is converted to an amount per unit area of the measured substrate. A detailed method for measuring the amount of dissolution will be described later. When the amount of dissolution of the hydrophilic polymer exceeds the above range, there is a concern that, in medical devices that are in contact with blood, the dissolved hydrophilic polymer is accumulated in the body of patients. When the molecular weight of the hydrophilic polymer exceeds 50,000, the polymer is not filtered by the kidneys and is not excreted from the body. Therefore, such accumulation is a particular concern. Furthermore, when the substrate is used as an artificial kidney, the artificial kidney is used for patients who have poor or no their renal function. Therefore, even when the molecular weight of the hydrophilic polymer is 50,000 or less, the accumulation in the body of patients is a concern. In addition, when the substrate is used as analytical instruments such as a protein chip or a biosensor, there is a concern that the dissolved hydrophilic polymer becomes an inhibiting factor in the analysis.

The condition for irradiating with radiation is preferably controlled as follows. In an aqueous solution of a hydrophilic polymer being in contact with a substrate, the maximum increasing value of ultraviolet absorption value in the wavelength range of 260 to 300 nm, the increase being caused by irradiating with radiation, is preferably 1 or less, more preferably 0.5 or less. Herein, the maximum increasing value of ultraviolet absorption value is defined as follows: Values are calculated by subtracting the ultraviolet absorption values of the aqueous solution of the hydrophilic polymer in the range of 260 to 300 nm before irradiating with radiation from the ultraviolet absorption values of the aqueous solution of the hydrophilic polymer in the same wavelength range after irradiating with radiation. Among the above values, the maximum value in the above wavelength range is defined as the maximum increasing value of the ultraviolet absorption value. Under some conditions for irradiating with radiation, the hydrophilic polymer is degraded to generate a substance absorbing light in the wavelength range of 260 to 300 nm and having a relatively high reactivity. In particular, in medical devices, the amount of such a substance is preferably small in terms of safety.

In the modified substrate, a surface hydrophilic polymer ratio is at least 20 weight percent. Herein, the surface hydrophilic polymer ratio is defined as a ratio represented by A/(A+B), wherein (A) is the weight of the monomer unit of the hydrophilic polymer on the surface of the modified substrate (the number of moles of the monomer unit × the molecular weight of the monomer unit) and (B) is the weight of the monomer unit of the polymer forming the substrate on the surface of the modified substrate (the number of moles of the monomer unit × the molecular weight of the monomer unit). This surface hydrophilic polymer ratio is a parameter representing the degree of hydrophilicity on the surface of the modified substrate.

The surface hydrophilic polymer ratio is measured by analyzing only the surface of the modified substrate, i.e., the depth profile of about 10 nm from the surface, by X-ray photoelectron spectrometry (ESCA). The surface hydrophilic polymer ratio is at least 20 weight percent, more preferably, at least 32 weight percent. When the surface hydrophilic polymer ratio is less than 20 weight percent, the effect at suppressing the adhesion of organic matter such as proteins,

or biogenic substances is decreased. This is because the hydrophilic polymer cannot cover the surface of the substrate, and therefore, the ratio of the substrate exposed on the surface of the modified substrate is increased.

In the modified substrate, a hydrophilic polymer is immobilized on the surface of the substrate, and in addition, for example, excessive crosslinking or degradation of the hydrophilic polymer is prevented. As a result, the adhesion of organic matter such as proteins, or biogenic substances can be suppressed. The modified substrate particularly has high hematologic compatibility. Specifically, in the modified substrate, the number of adhered human blood platelets is $10/4.3\times10^3\ \mu m^2$ or less. The number of adhered blood platelets is defined as follows: A modified substrate is brought into contact with blood for one hour. The number of blood platelets adhered on the surface of the modified substrate is represented as the number per $4.3\times10^3\ \mu m^2$ of the surface area of the modified substrate. Detailed methods for measuring the number of adhered blood platelets will be described later. When the number of adhered human blood platelets exceeds $10/4.3\times10^3\ \mu m^2$, hematologic compatibility is insufficient, and in addition, the effect at suppressing the adhesion of organic matter such as proteins, or biogenic substances is also insufficient.

Because of its high hematologic compatibility, the modified substrate can be preferably used as medical substrates. The medical substrates used in the present invention include substrates used in an artificial blood vessel, a catheter, a blood bag, a contact lens, an intraocular lens, auxiliary instruments for surgical operation, and a module for blood purification. In particular, the modified substrate is suitable for applications in which the substrate is brought into contact with a biogenic substance, for example, a module for blood purification such as an artificial kidney. Herein, the module for blood purification refers to a module having a function of circulating the blood in order to remove waste products or harmful substances from the blood in order to excrete them from the body. Examples of the module for blood purification include an artificial kidney and an adsorption column for exotoxins. The module for an artificial kidney includes a coil type, a flat plate type, and a hollow fiber membrane type. In terms of, for example, high processing efficiency, the hollow fiber membrane type is preferable.

Furthermore, medical substrates used for adsorbing and removing substances such as a cytokine, e.g., interleukin-6 (hereinafter abbreviated as IL-6), substances having an adverse effect on the living body, are known. Preferably, such medical substrates also have high hematologic compatibility. As a result of hydrophilization treatment performed on the surface of the substrate, the adhesion on the substrate of blood platelets or proteins related to clotting is suppressed. However, at the same time, the adsorption on the substrate of target substances to be removed such as IL-6 is also suppressed. The modified substrate can achieve high hematologic compatibility while maintaining the adsorption of a cytokine such as IL-6. Specifically, a modified substrate having high hematologic compatibility can be produced, while the adsorptivity to cytokine of the modified substrate is maintained so as to be at least 90% of the adsorptivity to cytokine of the substrate before modification. In the modified substrate, the adsorptivity to TL-6 is preferably at least 0.1 $ng/cm^2$. When the adsorptivity to IL-6 is within this range, the modified substrate can be preferably used as an adsorption column for IL-6.

Preferably, the modified substrate can also be used as, for example, separation membranes for water treatment, separation membranes of biogenic substances, instruments used for biological experiments, bioreactors, molecular motors, DDS, protein chips, DNA chips, biosensors, or components of analytical instruments, utilizing the feature in which the modified substrate suppresses the adhesion of biogenic substances. In addition, since the modified substrate includes a hydrophilic polymer having a low degree of three-dimensional crosslinking thereon, the modified substrate can be applied to a material that requires low frictionality.

In the present invention, the substrate represents a material to which hydrophilicity is provided. The substrate is composed of a polymeric material. Examples of the polymeric material include polysulfones, polystyrene, polyurethanes, polycarbonate, polymethylmethacrylate, polyethylene, polypropylene, polyvinylidene fluoride, polyacrylonitrile, polyesters, and polyamides. These polymeric materials may be used as a copolymer. Furthermore, carbon materials such as carbon fibers; carbon plates e.g., a glassy carbon plate and a carbon sheet; carbon nanotube; and fullerene; and composite materials including these carbon materials and a resin may also be used. Materials prepared by substituting a part of these materials with a functional group can also be applied as the substrate. The reaction mechanism to provide hydrophilicity using the carbon materials is not known exactly. It is not known whether the carbon materials directly react or a trace of impurities physically contained in the carbon materials reacts. However, the carbon materials can also make the substrate hydrophilic as in the polymeric materials. Examples of the form of a substrate include a fiber, a film, a resin, and a separation membrane. In the present method the substrate is a separation membrane system.

When a substrate is used as a medical substrate, a substrate is preferably composed of, for example, polyvinyl chloride; cellulose polymers; polystyrene; polymethylmethacrylate; polycarbonate; polysulfone polymers such as polysulfones and polyethersulfones; polyurethanes; polyacrylonitrile; and polyvinylidene fluoride. In particular, in the present method, polysulfone polymers are selected because polysulfone polymers are readily formed and separation membranes composed of polysulfone polymers have an excellent performance in terms of the permeation of a substance. The substrate of the method of the invention is a separation membrane system comprising polysulfone and polyvinylpyrrolidone.

Polysulfone polymers include aromatic rings, a sulfonyl group, and an ether group in the main chain. For example,

polysulfones represented by the following chemical formula (1) and/or (2) are preferably used. Symbol n in the formulae is preferably 50 to 80.

(1)

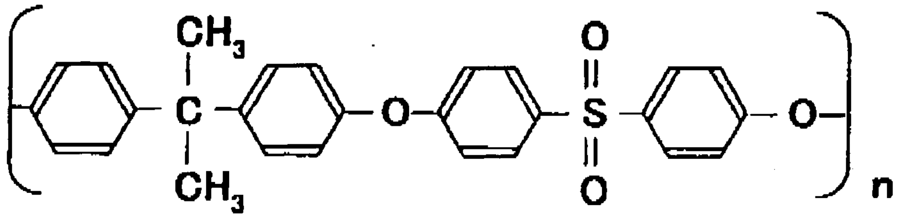

(2)

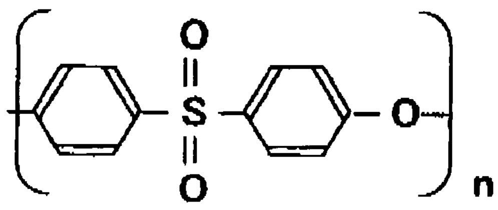

Examples of the polysulfones include Udel (registered trademark) polysulfone P-1700, P-3500 (from Teijin Amoco Engineering Plastics Limited); Ultrason (registered trademark) S3010 and S6010 (from BASF); Victrex (registered trademark) (from Sumitomo Chemical Co., Ltd.); Radel (registered trademark) A (from Teijin Amoco Engineering Plastics Limited); and Ultrason (registered trademark) E (from BASF). Although the polysulfones used in the present invention preferably include only the repeating unit represented by the above formula (1) and/or (2), the polysulfones may be copolymerized with other monomers so long as the advantage of the present invention is not impaired. The amount of the other copolymerization monomers is preferably 10 weight percent or less.

When a substrate is used as a medical substrate for adsorbing and removing a cytokine such as IL-6, the substrate is preferably composed of a hydrophobic polymer because such a polymer has a high adsorbing performance. Because of its high adsorbing performance, polymethylmethacrylate is particularly preferable.

In the present invention, a hydrophilic polymer refers to a polymer including a hydrophilic functional group in the main chain or the side chain of the polymer. Hydrophilic polymers having solubility in water at 25°C of, preferably, at least 0.001 weight percent, more preferably, at least 0.01 weight percent, and most preferably, at least 0.1 weight percent, are readily applied to the present technology. Examples of a hydrophilic polymer include polyvinylpyrrolidone, polyethylene glycol, polypropylene glycol, polyvinyl alcohol, polyethyleneimine, polyallylamines, polyvinylamine, polyvinyl acetate, polyacrylic acid, polyacrylamide, and copolymers and graft polymers of these and other monomers. Nonionic hydrophilic polymers such as polyalkylene glycols and polyvinylpyrrolidone provide an inhibiting effect of nonspecific adsorption. Cationic hydrophilic polymers such as polyethyleneimine provide an excellent inhibiting effect of adsorption of acidic substances such as an oxidized low-density lipoprotein (LDL). Anionic polymers such as dextran sulfate and polyvinyl sulfate provide an excellent inhibiting effect of adsorption of basic substances such as lysozyme. In terms of a high inhibiting effect of adsorption, polyvinylpyrrolidone is selected in the method of the invention.

The method for measuring the amount of hydrophilic polymer immobilized on the surface of the substrate is different depending on the kinds of substrate and hydrophilic polymer and the method is appropriately selected. Preferably, the amount of the hydrophilic polymer bonded on the modified substrate is directly measured. However, more simple methods may also be used. For example, the concentration of the hydrophilic polymer in an aqueous solution before irradiating with radiation may be compared with that in the aqueous solution after irradiating with radiation. Thus, the amount of decrease in the hydrophilic polymer in the aqueous solution is calculated. This amount may be defined as the amount of the immobilized hydrophilic polymer. In another simple method, the contact angle of the surface may be measured to estimate the amount of the immobilized hydrophilic polymer.

Also, polymers derived from the living body, for example, proteins are are hydrophilic polymers. Immobilization on the substrate of such a polymer derived from the living body can provide the substrate with a function of the polymer derived from the living body. Examples of the polymer derived from the living body include polymers having a sugar chain structure such as dextran and dextran sulfate, peptides, proteins, lipids, and composites such as polysaccharides.

The use of a plurality of hydrophilic polymers is also preferable. For example, when a nonionic hydrophilic polymer and a cationic hydrophilic polymer are used, the nonionic hydrophilic polymer provides an inhibiting effect of nonspecific adsorption, and in addition, the cationic hydrophilic polymer provides an excellent inhibiting effect of adsorption of acidic substances such as an oxidized low-density lipoprotein (hereinafter referred to as oxidized LDL). Thus, both advantages in the two hydrophilic polymers can be provided. When a nonionic hydrophilic polymer and an anionic

polymer are used, the nonionic hydrophilic polymer provides the inhibiting effect of nonspecific adsorption, and in addition, the anionic polymer provides an efficient inhibiting effect of adsorption of basic substances such as lysozyme. When a synthetic hydrophilic polymer and a hydrophilic polymer derived from the living body are used at the same time, a modified substrate having high hematologic compatibility and a function of the biopolymer can be provided. In order to immobilize a plurality of hydrophilic polymers, the hydrophilic polymers may be immobilized one after another. Alternatively, a mixture of a plurality of hydrophilic polymers may be immobilized at one time. This method is simple and more preferable.

The molecular weight of the hydrophilic polymer is preferably at least 100, more preferably, at least 500, and most preferably at least 1,000. The molecular weight of the hydrophilic polymer is preferably 50,000 or less.

Examples of the radiation are γ-ray and electron beams. Medical devices such as an artificial kidney require sterilization. In terms of low residual toxicity and convenience, recently, radiosterilization using γ-ray or an electron beam is often used. When the method of the present invention, which comprises irradiating with γ-ray, is used in medical substrates, sterilization and modification of a substrate can be achieved at the same time. In particular, the method of the present invention is preferably applied to an artificial kidney. In the artificial kidney, a wet type is mainly used in which the separation membrane is in a state containing water. Accordingly, the method of the present invention can be conveniently used by only replacing the water with an aqueous solution containing a hydrophilic polymer solution.

When sterilization and modification of a substrate are performed at the same time, the substrate is irradiated with radiation with an absorbed dose of at least 20 kGy. This is because an absorbed dose of at least 20 kGy is effective in order to sterilize, for example, a module for blood purification with γ-ray. However, when the absorbed dose is 20 kGy or more, the hydrophilic polymer is subjected to three-dimensional crosslinking or degraded, thereby decreasing hematologic compatibility. Therefore, in the present invention, an antioxidant is added. Specifically, the substrate is irradiated with radiation while the substrate is brought into contact with an aqueous solution containing a hydrophilic polymer and an antioxidant. The addition of the antioxidant provides the following features: Excessive crosslinking or degradation of the hydrophilic polymer can be prevented, while the hydrophilic polymer is immobilized, furthermore, sterilization can be performed at the same time.

The antioxidant according to the present invention refers to molecules that readily provide other molecules with electrons. When a hydrophilic polymer such as polyvinylpyrrolidone is subjected to a radical reaction with radiation, the antioxidant inhibits the reaction. Examples of antioxidants include water-soluble vitamins such as vitamin C; polyphenols; alcohols such as methanol, ethanol, propanol, ethylene glycol, and glycerin; saccharides such as glucose, galactose, mannose, and trehalose; inorganic salts such as sodium hydrosulfite, sodium pyrosulfite, and sodium dithionate; uric acid; cysteine; glutathione; and oxygen. Antioxidants may be used alone or in combination of two or more. When the method of the present invention is used in medical devices, the safety must be considered, and antioxidants having low toxicity are preferably used in such a case. In particular, in the method of the invention the alcohols and inorganic salts listed above are used.

The concentration of antioxidant in an aqueous solution is different depending on, for example, the kind of antioxidant and the exposure dose of radiation. An excessively low concentration of antioxidant causes three-dimensional crosslinking or degradation of the hydrophilic polymer to decrease hematologic compatibility. On the other hand, the addition of an excessive amount of antioxidant decreases the immobilization efficiency on the substrate. Therefore, sufficient hematologic compatibility is not achieved.

A method for producing a modified substrate of the present invention will now be described in detail with reference to an example using an antioxidant.

In a method for modifying the substrate, the substrate is irradiated with radiation while the substrate is brought into contact with an aqueous solution containing a hydrophilic polymer and an antioxidant. For example, when the substrate is a film, preferably, the substrate is irradiated with radiation while the substrate is immersed in an aqueous solution containing a hydrophilic polymer and an antioxidant. When the substrate is a hollow substrate such as a hollow fiber membrane and hydrophilicity should be provided on the inner surface of the hollow part, the aqueous solution is filled inside of the hollow part and then the substrate is preferably irradiated with radiation. Furthermore, when the substrate is disposed in a module, the aqueous solution is filled in the module and then the whole module is preferably irradiated with radiation. For example, in an artificial kidney, separation membranes are disposed in a module case. In such a case, an aqueous solution containing a hydrophilic polymer and an antioxidant is filled in the module and then the whole module may be irradiated with radiation. Alternatively, only the separation membranes may be irradiated with radiation while the separation membranes are immersed in the aqueous solution containing the hydrophilic polymer and the antioxidant. Subsequently, the separation membranes may be fitted in the module. Since modification and sterilization can be performed at the same time, more preferably, the aqueous solution containing the hydrophilic polymer and the antioxidant is filled in the module and then the whole module is irradiated with radiation.

Preferably, the substrate may be irradiated with radiation while the substrate is in a wet state with an aqueous solution containing a hydrophilic polymer and an antioxidant. Herein, the wet state refers to a state in which the aqueous solution used for immersing the substrate is removed but the substrate is not dried. Although the water content is not particularly limited, the substrate preferably contains at least one weight percent of water relative to the dry substrate.

In other words, the substrate is immersed in the aqueous solution and is then removed from the aqueous solution. Subsequently, the substrate may be irradiated with radiation. Alternatively, the aqueous solution is filled in the module including the substrate and most of the aqueous solution is then discharged from the module with, for example, a nitrogen gas jet. Subsequently, the module may be irradiated with radiation.

In another method, the substrate is immersed in an aqueous solution of a hydrophilic polymer in advance such that the surface of the substrate is coated with the hydrophilic polymer. Subsequently, the substrate may be irradiated with $\gamma$-ray while the substrate is immersed in a solution containing an antioxidant. This method can also make the surface of the substrate hydrophilic efficiently. The area to which the hydrophilic polymer is provided can be variously controlled according to the kind of substrate and the method of modification. For example, in a substrate used as a hollow fiber membrane, an aqueous solution containing a hydrophilic polymer is introduced to the inside of the hollow fiber membrane and the hollow fiber membrane is then irradiated with radiation. In such a case, the hydrophilic polymer can be immobilized on the inner surface of the hollow fiber membrane. For example, this method is preferably applied to an artificial kidney in which the substrate is used such that blood flows only on the inner surface thereof. In addition to the inner surface, when hydrophilization needs to be performed on the outer surface of the hollow fiber membrane, the aqueous solution containing the hydrophilic polymer is brought into contact with the outer surface of the hollow fiber membrane. For example, when hollow fiber membranes are disposed in a module case, the aqueous solution containing the hydrophilic polymer is filled in the clearance formed between the hollow fiber membranes and the module case.

In a substrate used as a separation membrane, an aqueous solution containing a hydrophilic polymer is filled while the solution is filtered through the membrane. Since the hydrophilic polymer is concentrated on the surface of the membrane, this method is effective at making the surface more hydrophilic. In such a case, when a polymer that does not readily permeate through the membrane, for example, a high-molecular weight hydrophilic polymer, is used as the hydrophilic polymer, the hydrophilic polymer is further concentrated on the surface of the membrane to provide a higher effect.

In contrast, when a low-molecular weight hydrophilic polymer is used, hydrophilization treatment can be performed on the inside of the membrane. For example, in a membrane used for separating biogenic substances and recovering a part of the substances by filtering or dialysis, i.e., a separation membrane of biogenic substances, even when only the surface of the membrane is subjected to hydrophilization, the adsorption of the biogenic substances at the inside of the membrane cannot be suppressed. Accordingly, in an embodiment of the separation membrane of biogenic substances, hydrophilization treatment is preferably performed on the inside of the membrane.

In the present invention, a plurality of substrates are irradiated with radiation at the same time, while a system including the plurality of the substrates is brought into contact with an aqueous solution containing a hydrophilic polymer and an antioxidant. Thus, a plurality of substrates can be modified at one time. In particular, when the plurality of substrates are composed of different materials, this method provides a significant effect. In a known method for modification, it is difficult to modify a plurality of substrates composed of different materials at the same time because the conditions for modifying each substrate significantly depend on the kinds of the substrates.

Herein, the system including a plurality of substrates refers to, for example, a separation membrane system including port elements, separation membranes, and a circuit. For example, modules for blood purification such as an artificial kidney and an adsorption column for exotoxins include a plurality of substrates such as a catheter, a blood circuit, a chamber, an inlet port element and an outlet port element of a module, and separation membranes, the substrates being composed of different materials. In the present invention, all or a part of the substrates can be modified at the same time. Preferably, at least a part of the port elements, the separation membranes, and the circuit is modified. For example, in an artificial kidney system, an inlet port element of a module, an outlet port element of the module, and a blood circuit are connected to a hollow fiber membrane module. An aqueous solution of a hydrophilic polymer is then introduced from the blood circuit to fill the entire system with the solution. Subsequently, the entire system is irradiated with radiation in this state.

Various methods for producing a module for blood purification are known depending on the application. The methods are broadly divided into the steps of producing separation membranes for blood purification and the steps of fitting the separation membranes in the module.

An example of a method for producing a hollow fiber membrane module used in an artificial kidney will now be described. A method for producing a hollow fiber membrane fitted in the artificial kidney includes the following method. A stock solution is prepared by dissolving a polysulfone and polyvinylpyrrolidone in a good solvent or a mixed solvent containing a good solvent. The concentration of the polymer is preferably 10 to 30 weight percent, more preferably, 15 to 25 weight percent. The ratio by weight of the polysulfone to the polyvinylpyrrolidone is preferably 20:1 to 1:5, more preferably, 5:1 to 1:1. N,N-dimethylacetamide, dimethylsulfoxide, dimethylformamide, and N-methylpyrrolidone, and dioxane are preferably used as the good solvent. The stock solution is discharged from an outer tube of a double-annular spinneret to run through a dry step. Subsequently, the stock solution is led to a coagulation bath. An injection liquid or a gas to form a hollow part is discharged from an inner tube of the double-annular spinneret. In this process, the humidity in the dry step affects the characteristics of the membrane. Therefore, moisture may be supplied from the outer surface

of the membrane while the stock solution runs through the dry step in order to accelerate a phase separation behavior in the vicinity of the outer surface. As a result, the diameter of the opening is increased. Thus, permeation resistance and diffusion resistance when used for dialysis can be decreased. However, when the relative humidity is excessively high, coagulation of the stock solution at the outer surface becomes dominant. As a result, the diameter of the opening is decreased. Accordingly, permeation resistance and diffusion resistance when used for dialysis are increased. Therefore, the relative humidity is preferably 60% to 90%. In terms of process suitability, the composition of the injection liquid preferably includes the solvent used to prepare the stock solution as a basic component. Regarding the concentration of the injection liquid, for example, when dimethylacetamide is used, an aqueous solution having a concentration of preferably 45 to 80 weight percent, more preferably, 60 to 75 weight percent is used.

Although a method for fitting hollow fiber membranes in a module is not particularly limited, an example of the method is as follows. Firstly, hollow fiber membranes are cut so as to have a desired length. A required number of the hollow fiber membranes are bundled to put in a cylindrical case. Subsequently, both ends are closed with temporal caps. A potting agent is added in both ends of the hollow fiber membranes. Preferably, the potting agent is added while the module is rotated with a centrifuge because the potting agent can be filled uniformly. After the potting agent is solidified, both ends are cut such that both ends of the hollow fiber membranes are opened, thus producing a hollow fiber membrane module.

Fig. 1 shows an example of the basic structure of an artificial kidney system using a hollow fiber membrane module produced by the above method. A bundle of hollow fiber membranes 5 is inserted in a cylindrical plastic case 7. A resin 10 seals both ends of the hollow fibers. The case 7 includes an inlet 8 and an outlet 9 for dialysate. For example, dialysate, physiological saline, or filtered water flows in the outside of the hollow fiber membranes 5. An inlet port element 1 and an outlet port element 2 are disposed at the ends of the case 7. Blood 6 is introduced from a blood inlet 3 disposed in the inlet port element 1, and is introduced to the inside of the hollow fiber membranes 5 by the port element 1 having a funnel shape. The blood 6 filtered with the hollow fiber membranes 5 is collected by the outlet port element 2 to discharge from a blood outlet 4. The blood inlet 3 and the blood outlet 4 are connected to a blood circuit 11.

The present invention will now be described with reference to Examples. The present invention is not limited by the Examples. Examples 4 to 8 and 13 to 15 are included for information only; they do not illustrate the invention of claim 1.

1. Methods for preparing substrates (Preparation of polysulfone film 1)

Polysulfone (Udel (registered trademark) P-3500 from Teijin Amoco Engineering Plastics Limited) (10 parts by weight) was added to N,N'-dimethylacetamide (80 parts by weight) and allowed to dissolve at room temperature. Thus, a membrane stock solution was prepared. A glass plate was heated with a hot-plate such that the surface temperature of the glass plate was 100°C. The membrane stock solution was cast such that the thickness was 203 μm. The surface temperature was measured with a contact type thermometer. After the casting, the membrane was left to stand for 5 minutes on the hot-plate to evaporate the solvent. Subsequently, the whole glass plate was immersed in a water bath to prepare a polysulfone film 1. The purpose of the immersion in the water bath is to allow the polysulfone film to be peeled readily from the glass plate.

(Preparation of hollow fiber membrane module 1)

Polysulfone (Udel (registered trademark) P-3500 from Teijin Amoco Engineering Plastics Limited). (18 parts by weight) and polyvinylpyrrolidone (K30 from BASF) (9 parts by weight) were added to a mixed solvent containing N,N'-dimethylacetamide (72 parts by weight) and water (1 part by weight). The mixture was heated at 90°C for 14 hours to dissolve the polymers. Thus, a membrane stock solution was prepared. The membrane stock solution was discharged from an outer tube of an orifice type double-cylindrical spinneret having an outer diameter of 0.3 mm and an inner diameter of 0.2 mm. A core liquid containing N,N'-dimethylacetamide (58 parts by weight) and water (42 parts by weight) was discharged from an inner tube. The discharged membrane stock solution was passed through a dry step having a length of 350 mm and was then introduced in a 100% water coagulation bath. Thus, a hollow fiber was prepared.

The resultant 10,000 hollow fibers were inserted in a cylindrical plastic case as shown in Fig. 1, which includes an inlet and an outlet for dialysate. Both ends of the membranes were sealed with a resin to prepare a hollow fiber membrane module 1 for an artificial kidney having an effective membrane area of 1.6 $m^2$.

(Preparation of hollow fiber membrane module 2)

Isotactic-polymethylmethacrylate (5 parts by weight) and syndiotactic-polymethylmethacrylate (20 parts by weight) were added to dimethylsulfoxide (75 parts by weight). The mixture was heated to dissolve the polymers. Thus,

a membrane stock solution was prepared. The membrane stock solution was discharged from an outer tube of an orifice type double-cylindrical spinneret. The discharged membrane stock solution was passed through air for 200 mm and was then introduced in a 100% water coagulation bath. Thus, a hollow fiber was prepared. In this process, dry nitrogen was discharged from an inner tube as an inside injection gas. The resultant hollow fiber had an inner diameter of 0.2 mm and a thickness of 0.03 mm. A hollow fiber membrane module 2 having an effective membrane area of 1.6 $m^2$ was prepared using the resultant 10,000 hollow fibers, as in the hollow fiber membrane module 1.

2. Measuring method

(1) Measurement of the soluble hydrophilic polymer ratio

A measurement sample was dried and the dry weight was measured. Subsequently, the sample was dissolved in a solvent that can dissolve both the substrate and the hydrophilic polymer. A solvent that dissolves the hydrophilic polymer but does not dissolve the substrate was added to the resultant solution. As a result of this operation, the substrate and the hydrophilic polymer immobilized on the substrate were precipitated, whereas a soluble hydrophilic polymer remained dissolved. The amount of hydrophilic polymer in the supernatant was quantitatively determined by high performance liquid chromatography (HPLC). Thus, the weight of soluble hydrophilic polymer per unit weight of the measurement sample could be calculated. On the other hand, the elemental analysis of the measurement sample provided the weight of total hydrophilic polymer per unit weight of the measurement sample. The soluble hydrophilic polymer ratio was calculated by dividing the weight of soluble hydrophilic polymer per unit weight of the measurement sample by the weight of total hydrophilic polymer per unit weight of the measurement sample.

When polyvinylpyrrolidone was used as the hydrophilic polymer and Udel (registered trademark) P-3500 was used as the substrate, the soluble hydrophilic polymer ratio was measured as follows. A dry measurement sample was dissolved in N-methyl-2-pyrrolidone such that the concentration of the solution was 2.5 weight percent. Water (1.7 fold by volume) was added dropwise to the solution while the solution was stirred, thereby precipitating the substrate polymer. In this process, the water should not be added at once because the polysulfone is precipitated while the polysulfone becomes entangled with soluble polyvinylpyrrolidone. Attention should be paid because an accurate measurement may be impossible in such a case. The soluble polyvinylpyrrolidone was included in the solution with the dispersed fine polysulfone particles. The solution was filtered with a nonaqueous filter (from Tosoh Corporation, diameter 2.5 μm) for HPLC to remove the fine polysulfone particles in the solution. Subsequently, polyvinylpyrrolidone in the filtrate was quantitatively determined by HPLC under the following conditions.

Apparatus: Waters, GPC-244
Column: TSK-gel GMPWXL, 2 columns
Solvent: Water-based, 0.1 M ammonium chloride, 0.1 N ammonia, pH 9.5
Flow rate: 1.0 mL/min.
Temperature: 23°C

The weight of soluble polyvinylpyrrolidone per unit weight of the measurement sample was calculated from the amount of polyvinylpyrrolidone in the filtrate. This weight was divided by the weight of total polyvinylpyrrolidone per unit weight of the measurement sample, which was determined by elemental analysis. Thus, the soluble polyvinylpyrrolidone ratio was determined.

(2) Dissolution test of hydrophilic polymer

An aqueous solution of a hydrophilic polymer in which a measurement sample was immersed was removed. Subsequently, the measurement sample was immersed in water at 37°C for 4 hours. The volume of water was 0.25 $mL/cm^2$ relative to the area of the surface of the modified substrate. Thus, the amount of dissolved hydrophilic polymer was quantitatively determined.

When the hollow fiber membrane module 1 was used as the measurement sample, the amount of dissolution was measured as follows. The blood side of the hollow fiber membrane module 1 was washed with 700 mL of ultrapure water at room temperature, and the dialysate side thereof was washed with 2,500 mL of ultrapure water at room temperature. The blood side was then washed again with 300 mL of ultrapure water at room temperature to wash away hydrophilic polymers originally included in the filling fluid. Subsequently, the blood side was perfused with 4,000 mL of ultrapure water heated at 37°C for 4 hours at a flow rate of 200 mL/min. Subsequently, the perfusate was concentrated by 200 fold to measure by gel permeation chromatography (GPC).. The total amount of hydrophilic polymer dissolved in the perfusate was calculated from the analytical value. When the hydrophilic polymer was polyvinylpyrrolidone, the measurement conditions for GPC were as follows. A GMPWXL column was used, the flow rate was 0.5 mL/min., a mixed

solvent of methanol containing 0.1 N lithium nitrate : water = 1 : 1 (volume ratio) was used as the solvent, and the column temperature was 40°C. Polyvinylpyrrolidone K90 (from BASF) was used for a calibration curve of the concentration of polyvinylpyrrolidone.

(3) Measurement of maximum increasing value of ultraviolet absorption value

An ultraviolet absorption value of an aqueous solution of a hydrophilic polymer being in contact with a measurement sample was measured before and after irradiating with radiation. The ultraviolet absorption value was measured in a wavelength range of 260 to 300 nm. An aqueous solution (about 3 mL) for measurement was prepared in a quartz cell having an optical path length of 1 cm. The ultraviolet absorption value was measured with a spectrophotometer U-2000 (from Hitachi, Ltd.) at room temperature. The increasing value of ultraviolet absorption value was calculated by subtracting the ultraviolet absorption value measured before irradiating with radiation from the ultraviolet absorption value measured after irradiating with radiation. The maximum increasing value in the wavelength range of 260 to 300 nm was defined as the maximum increasing value of ultraviolet absorption value.

When a hollow fiber membrane module was used as the measurement sample and an aqueous solution of a hydrophilic polymer was filled in the blood side, after irradiating with radiation, only the aqueous solution dripping by free fall was sampled. However, when the aqueous solution of the hydrophilic polymer was filled in the blood side, the solution was then discharged by, for example, blowing, and the substrate was irradiated with radiation in a wet state, the aqueous solution might not drip by free fall. In such a case, water is filled in the module again, and the module is left to stand at room temperature for at least one hour. Subsequently, water at the blood side dripping by free fall may be sampled.

When a substrate other than a hollow fiber membrane module is irradiated with radiation in a wet state, the substrate is immersed in water of 0.1 $mL/cm^2$ at room temperature for one hour. Subsequently, the measurement is performed using the water, and the measured value is multiplied by 20. The resultant value is used. In the above hollow fiber membrane module, the volume of filling fluid at the blood side relative to the inner surface area, that is, the bath ratio, is 0.005 $mL/cm^2$. The above calculation indicates that the bath ratio is converted so as to correspond with the above value. If the substrate cannot be immersed in the water volume of 0.1 $mL/cm^2$, water may be appropriately added to perform the measurement. Subsequently, the bath ratio is converted so as to correspond with 0.005 $mL/cm^2$.

(4) Measurement of surface hydrophilic polymer ratio

The hydrophilic polymer ratio on the surface was measured by X-ray photoelectron spectrometry (ESCA). A measurement apparatus ESCALAB220iXL was used and a sample was prepared in the apparatus. In the measurement, the angle of a detector to the angle of incidence of X-ray was 90 degrees. In a film sample, the surface of the film on the glass used for casting was measured. In a hollow fiber membrane sample, the hollow fiber membrane was cut with a single edged knife to form a semicylindrical shape and the inner surface of the hollow fiber membrane was measured. The measurement sample was rinsed with ultrapure water and was then dried at room temperature and at 0.5 Torr for 10 hours. Subsequently, the sample was used for the measurement.

When polyvinylpyrrolidone was used as the hydrophilic polymer and Udel (registered trademark) P-3500 was used as the substrate, the surface polyvinylpyrrolidone ratio was calculated as follows. The amount of nitrogen (a) on the surface and the amount of sulfur (b) on the surface were calculated from the integrated intensity of C1s, N1s, and S2p spectra, which were obtained by ESCA, using a relative sensitivity coefficient provided from the apparatus. The surface polyvinylpyrrolidone ratio was calculated by the following formula:

$$\text{Surface polyvinylpyrrolidone ratio (weight percent)} = a\times 100/(a\times 111+b\times 442)$$

(5) Measurement of immobilization density of polyethylene glycol

A hollow fiber after irradiating with radiation was immersed in distilled water at 37°C for one hour. The volume of the distilled water was 1 L per 1 $m^2$ of the surface area of the substrate. The hollow fiber was washed while distilled water was changed until the amount of polyethylene glycol dissolved into the distilled water was 1 mg or less. Thus, polyethylene glycol that is not immobilized on the substrate was removed. The washed substrate was dried at 50°C and at 0.5 Torr for 10 hours. In a test tube, 10 to 100 mg of the dry substrate was prepared. A mixed solution (2 mL) containing acetic anhydride and para-toluenesulfonic acid was added to the substrate to acetylate the mixture at 120°c for about one hour. After cooling, the wall was washed with 2 mL of purified water. Subsequently, 20% sodium hydrogencarbonate was added to the mixture to neutralize. The neutralized solution was extracted with trichloromethane (5 mL). The extract

was analyzed by gas chromatography (hereinafter abbreviated as GC). The analytical conditions for GC were as follows. The amount of polyethylene glycol immobilized on the substrate was determined using a calibration curve prepared in advance.

(Analytical conditions for GC)

Apparatus: Shimadzu GC-9A
Column: Supelcowax-10, 60 m × 0.75 mm I.D.
Carrier gas: Helium
Detector: Flame-ionization detector (FID) ($H_2$ inlet: 0.7 kg/cm$^2$, Air inlet: 0.6 kg/cm$^2$, Temperature: 200°C)
Column temperature: 80°C, holding for 5 min.-(20 min.)-200°C, holding for 5 min.
Injector temperature: 200°C

(6) Measurement of contact angle

The contact angle was measured with a contact angle meter CA-D from Kyowa Interface Science Co., Ltd. The measurement was performed in a room where the room temperature was controlled at 25°C.

(7) Method of adhering test of rabbit blood platelets on film

A film for measurement was disposed on the bottom of a cylindrical polystyrene tube having a diameter of 18 mm. The cylindrical tube was filled with physiological saline. If contaminations, flaws, fold lines, or the like are disposed on the surface of the film, blood platelets are adhered on such areas. Attention should be paid because an accurate evaluation may be impossible in such a case. A blood sample containing an aqueous solution of 3.2% trisodium citrate dihydrate and fresh rabbit blood at a volume ratio of 1:9 was subjected to centrifugal separation at 1,000 rpm for 10 minutes to recover the supernatant (referred to as blood plasma 1). After the supernatant was recovered, the resultant blood was subjected to centrifugal separation again at 3,000 rpm for 10 minutes to recover the supernatant (referred to as blood plasma 2). The blood plasma 1 was diluted by adding the blood plasma 2 (the concentration of blood platelets in the blood plasma 2 was lower than that in the blood plasma 1) to prepare a platelet-rich plasma (hereinafter referred to as PRP) having $20\times10^6$ /mL of blood platelets. The physiological saline prepared in the cylindrical tube was removed and 1.0 mL of the PRP was then added in the cylindrical tube. The cylindrical tube was shaken at 37°C for one hour. Subsequently, the measurement film was washed three times with physiological saline. The blood component was fixed with an aqueous solution of 3% glutaraldehyde. The film was washed with distilled water and was then dried under a reduced pressure for at least 5 hours.

The film was adhered on a specimen support for a scanning electron microscope with a double-sided adhesive tape. A thin film composed of Pt-Pd was deposited on the surface of the film by sputtering to prepare a sample. The surface of the sample was observed with a scanning electron microscope (S800 from Hitachi, Ltd.). Since the blood readily retained in the portions of the film being in contact with the cylindrical tube, the central part of the film was mainly observed at a magnification ratio of 3,000 to count the number of adhered blood platelets found per one field of view ($1.12\times10^3$ $\mu m^2$). The average number of adhered blood platelets in 10 different fields of view in the vicinity of the center of the film was calculated. The number of adhered blood platelets (number/$1.0\times10^3$ $\mu m^2$) was calculated by dividing the above average number of adhered blood platelets by 1.12.

(8) Method of adhering test of human blood platelets on film

A film for measurement was fixed on a polystyrene circular plate having a diameter of 18 mm with a double-sided adhesive tape. If contaminations, flaws, fold lines, or the like are disposed on the surface of the film, blood platelets are adhered on such areas. Attention should be paid because an accurate evaluation may be impossible in such a case. The circular plate was fitted in a Falcon (registered trademark) tube (18 mm in diameter, No. 2051), which was cut in a tubular shape, such that the surface having the film thereon was disposed at the inside of the cylinder. The clearance was filled with Parafilm. The inside of this cylindrical tube was washed with physiological saline and was then filled with physiological saline. Human venous blood was collected and heparin was then added to the blood immediately so as to have a concentration of 50 U/mL. The physiological saline in the cylindrical tube was removed. Subsequently, 1.0 mL of the blood was filled in the cylindrical tube within 10 minutes from the collection. The cylindrical tube was shaken at 37°C for one hour. Subsequently, the measurement film was washed with 10 mL of physiological saline. The blood component was fixed with physiological saline containing 2.5% glutaraldehyde. The film was washed with 20 mL of

distilled water. The washed film was then dried at room temperature under a reduced pressure of 0.5 Torr for 10 hours. A thin film composed of Pt-Pd was then deposited on the surface of the film by sputtering to prepare a sample. The surface of the sample was observed with a field emission scanning electron microscope (S800 from Hitachi, Ltd.) at a magnification ratio of 1,500 to count the number of adhered blood platelets found per one field of view ($4.3 \times 10^3$ $\mu m^2$). The average number of adhered blood platelets in 10 different fields of view in the vicinity of the center of the film was calculated. The average number of adhered blood platelets was defined as the number of adhered blood platelets (number/$4.3 \times 10^3$ $\mu m^2$).

(9) Method of adhering test of rabbit blood platelets on hollow fiber membrane

Thirty hollow fiber separation membranes were bundled. Both ends of the membranes were fixed in a glass tube module case with an epoxy-based potting agent such that the hollow parts of the hollow fibers were not clogged. Thus, a mini module having a diameter of about 7 mm and a length of about 10 cm was prepared. A blood inlet of the mini module was connected to a dialysate outlet thereof with a silicone tube. In order to wash the hollow fibers and the inside of the module, 100 mL of distilled water was allowed to flow from a blood outlet at a flow rate of 10 mL/min. Physiological saline was then filled, and a dialysate inlet and the outlet were closed with caps. Subsequently, physiological saline was supplied from the blood inlet at a flow rate of 0.59 mL/min. for two hours to perform priming. A blood sample containing an aqueous solution of 3.2% trisodium citrate dihydrate and fresh rabbit blood at a volume ratio of 1:9 was prepared. Seven milliliters of the blood sample was perfused at a flow rate of 0.59 mL/min. for one hour. Subsequently, the membranes were washed with physiological saline using a 10-mL syringe. An aqueous solution of 3% glutaraldehyde was filled in the inside of the hollow fibers and the dialysate side. The module was left to stand at least one night to perform glutaraldehyde fixation. Subsequently, the glutaraldehyde was washed with distilled water. A hollow fiber membrane was cut out from the mini module and was dried under a reduced pressure for at least 5 hours. The hollow fiber membrane was adhered on a specimen support for a scanning electron microscope with a double-sided adhesive tape. The membrane was then sliced in the longitudinal direction so as to expose the inner surface. A thin film composed of Pt-Pd was deposited on the sample by sputtering. The inner surface of the sample was observed with a scanning electron microscope (S800 from Hitachi, Ltd.) at a magnification ratio of 3,000 to count the number of adhered blood platelets found per one field of view ($1.12 \times 10^3$ $\mu m^2$). The average number of adhered blood platelets in 10 different fields of view was calculated. The number of adhered blood platelets (number/$1.0 \times 10^3$ $\mu m^2$) was calculated by dividing the above average number of adhered blood platelets by 1.12.

(10) Method of adhering test of human blood platelets on hollow fiber membrane

A hollow fiber membrane was fixed on a polystyrene circular plate having a diameter of 18 mm with a double-sided adhesive tape. The adhered hollow fiber membrane was cut with a single edged knife to form a semicylindrical shape, thereby exposing the inner surface of the hollow fiber membrane. If contaminations, flaws, fold lines, or the like are disposed on the inner surface of the hollow fiber, blood platelets are adhered on such areas. Attention should be paid because an accurate evaluation may be impossible in such a case. The circular plate was fitted in a Falcon (registered trademark) tube (18 mm in diameter, No. 2051), which was cut in a tubular shape, such that the surface having the hollow fiber membrane thereon was disposed at the inside of the cylinder. The clearance was filled with Parafilm. The inside of this cylindrical tube was washed with physiological saline and was then filled with physiological saline. Human venous blood was collected and heparin was then added to the blood immediately so as to have a concentration of 50 U/mL. The physiological saline in the cylindrical tube was removed. Subsequently, 1.0 mL of the blood was filled in the cylindrical tube within 10 minutes from the collection. The cylindrical tube was shaken at 37°C for one hour. Subsequently, the hollow fiber membrane was washed with 10 mL of physiological saline. The blood component was fixed with physiological saline containing 2.5% glutaraldehyde. The hollow fiber membrane was washed with 20 mL of distilled water. The washed hollow fiber membrane was then dried at room temperature under a reduced pressure of 0.5 Torr for 10 hours. The film was adhered on a specimen support for a scanning electron microscope with a double-sided adhesive tape. A thin film composed of Pt-Pd was then deposited on the surface of the hollow fiber membrane by sputtering to prepare a sample. The inner surface of the hollow fiber membrane was observed with a field emission scanning electron microscope (S800 from Hitachi, Ltd.) at a magnification ratio of 1,500 to count the number of adhered blood platelets found per one field of view ($4.3 \times 10^3$ $\mu m^2$). The average number of adhered blood platelets in 10 different fields of view in the vicinity of the center of the hollow fiber in the longitudinal direction was calculated. The average number of adhered blood platelets was defined as the number of adhered blood platelets (number/$4.3 \times 10^3$ $\mu m^2$). This was because the blood readily retained at the end portions of the hollow fiber in the longitudinal direction.

(11) Method of adhering test of human blood platelets in blood circuit for artificial kidney

A blood circuit for an artificial kidney was finely cut into small pieces of about 0.1 g. (If a mesh part was used, the weight was about 0.01 g.) An adhering test of human blood platelets was performed using the small pieces as in the above item (9).

In the adhering tests of blood platelets described in the above items (7) to (11), in order to confirm whether the tests are adequately performed or not, a positive control and a negative control were added in each test as a benchmark. The positive control was a known sample in which a large amount of blood platelets can be adhered. In contrast, the negative control was a known sample in which a small amount of blood platelets is adhered. In the adhering tests of human blood platelets, a sample having a number of adhered blood platelets of at least 40 ($/4.3\times10^3$ $\mu m^2$) under the above experimental conditions was used as the positive control. In addition, a sample having a number of adhered blood platelets of up to 5 ($/4.3\times10^3$ $mm^2$) was used as the negative control. In the adhering tests of rabbit blood platelets, a sample having a number of adhered blood platelets of at least 30 ($/1.0\times10^3$ $\mu m^2$) was used as the positive control. In addition, a sample having a number of adhered blood platelets of up to 5 ($/1.0\times10^3$ $\mu m^2$) was used as the negative control. In the following Examples, a hollow fiber membrane used in an artificial kidney Filtryzer BG-1.6U from Toray Industries, Inc. was used as the positive control. A hollow fiber membrane used in an artificial kidney PS-1.6UW from Kawasumi Laboratories, Inc. was used as the negative control. After a test, when the number of blood platelets adhered on the positive control was the above value or more, and in addition, the number of blood platelets adhered on the negative control was the above value or less, the measurement values could be used. When the number of blood platelets adhered on the controls was not within the above ranges, the test was performed again. In such a case, the freshness of the blood might be insufficient or the blood might be excessively activated.

(12) Adsorption test of IL-6

The same thirty hollow fiber separation membranes as used in the above hollow fiber membrane module 2 were bundled. Both ends of the membranes were fixed in a glass tube module case with an epoxy-based potting agent such that the hollow parts of the hollow fibers were not clogged. Thus, a mini module having a diameter of about 7 mm and a length of about 10 cm was prepared. A blood inlet of the mini module was connected to a dialysate outlet thereof with a silicone tube. In order to wash the hollow fibers and the inside of the module, 100 mL of distilled water was allowed to flow from a blood outlet at a flow rate of 10 mL/min. Subsequently, an aqueous solution of PBS (Dulbecco PBS (-) from Nissui Pharmaceutical Co., Ltd.) was filled, and a dialysate inlet and the outlet were closed with caps.

IL-6 was added to 10 mL of human plasma so as to have a concentration of 1 ng/mL (referred to as liquid 1). The dialysate inlet and the dialysate outlet were closed with the caps, and the inlet of the blood side was connected to the outlet of the blood side with a silicone tube. Perfusion was performed at 37°C for 4 hours with the liquid 1 at a flow rate of 1 mL/min. The IL-6 was quantitatively determined before and after the perfusion. The adsorptivity on the substrate was calculated from the decrease in the IL-6.

(13) Method of adsorptive removal test of oxidized LDL

(a) Preparation of antioxidized LDL antibody

Antioxidized LDL antibody specimens prepared by Itabe et al. (H. Itabe et al., J. Biol. Chem. Vol. 269: p. 15274, 1994) were used. Specifically, mice were immunized by injecting a human atherosclerotic lesion homogenate. The hybridomas were prepared from the spleen cells of the mice, followed by screening those that were allowed to react with LDL that had been treated with copper sulfate. Thus, the antioxidized LDL antibody was prepared. The resultant antibody was classified as mouse IgM, and was not allowed to react with native LDL, acetylated LDL, or malondialdehyde-treated LDL. On the other hand, the antioxidized LDL antibody was allowed to react with some peroxidation products of phosphatidylcholine, including aldehyde derivatives and hydroperoxides of phosphatidylcholine. The antioxidized LDL antibody was dissolved in a 10 mM borate buffer solution (pH 8.5) containing 150.mM NaCl. The solution (protein concentration 0.60 mg/mL) was used as specimens.

(b) Preparation of oxidized LDL

A commercial LDI. (from Funakoshi Co., Ltd.) was desalinated and was then diluted with a phosphate buffer solution (hereinafter abbreviated as PBS) so as to have a concentration of 0.2 mg/mL. Subsequently, 2 weight percent of a 0.5 mM aqueous solution of copper sulfate was added to the solution. The solution was allowed to react at 37°C for 5 hours. A 25 mM ethylenediaminetetraacetic acid (EDTA) solution and 10 weight percent sodium azide were added to the resultant solution such that the concentration of the EDTA was 1 weight percent and the concentration of the

sodium azide was 0.02 weight percent. This solution was used as an oxidized LDL specimen.

(c) Determination of the concentration of oxidized LDL

The above antioxidized LDL antibody was diluted with PBS so as to have a concentration of 5 μg/mL. The solution was dispensed to a 96-well plate at a rate of 100 μL/well. The plate was shaken at room temperature for two hours. Subsequently the plate was left to stand at 4°C for at least one night to allow the antibody to be adsorbed on the walls.

The antibody solution was removed from the wells. A tris-HCl buffer solution (pH 8.0) containing 1% bovine serum albumin (BSA, Fraction V from Seikagaku Corporation) was dispensed at a rate of 200 μL/well. The plate was shaken at room temperature for two hours to block the walls. The BSA solution was then removed from the wells. Blood plasma containing the oxidized LDL was dispensed at a rate of 100 μL/well. Standard solutions used for plotting a calibration curve were dispensed at a rate of 100 μL/well. The plate was shaken at room temperature for 30 minutes and was then left to stand at 4°C for one night.

The temperature of the specimens was increased to room temperature and the solution was removed from the wells. The wells were washed three times with a tris-HCl buffer solution (pH 8.0) containing 0.05% Tween (registered trademark)-20. A solution of sheep anti-apoB antibody diluted with a 2,000-fold volume of PBS was dispensed in each washed well at a rate of 100 μL/well. The plate was shaken at room temperature for two hours and the anti-apoB antibody was removed from the wells. The wells were washed three times with a tris-HCl buffer solution (pH 8.0) containing 0.05% Tween-20. Subsequently, alkaline phosphatase-conjugated donkey anti-sheep IgG antibody diluted with a 2,000-fold volume of a tris-HCl buffer solution (pH 8.0) containing 2% Block Ace (from Dainippon Pharmaceutical Co., Ltd.) was dispensed in each washed well at a rate of 100 μL/well. The plate was shaken at room temperature for two hours. Subsequently, the conjugated antibody was removed from the wells. The wells were washed three times with a tris-HCl buffer solution (pH 8.0) containing 0.05% Tween-20. The wells were further washed two times with a tris-HCl buffer solution (pH 8.0). Subsequently, a solution (0.0005 M $MgCl_2$, 1 M diethanolamine buffer solution, pH 9.8) of p-nitrophenyl phosphate (1 mg/mL) was dispensed at a rate of 100 μL/well. The plate was allowed to react at room temperature for an adequate period of time. Subsequently, the absorbance at the wavelength of 415 nm was measured with a plate reader. The calibration curve was plotted using the results with the standard solutions to determine the concentration of the oxidized LDL.

(d) Measurement of adsorptive removal ratio of oxidized LDL

The above oxidized LDL was added to blood plasma of a normal healthy subject (30-years old Japanese, LDL (β lipoprotein) concentration 275 mg/dL, HDL-cholesterol concentration 70 mg/dL) so as to have a concentration of 2 μg/mL.

Seventy hollow fiber membranes were bundled and were inserted in a glass tube module case having a diameter of about 7 mm and a length of 12 cm. Both ends of the hollow fiber membranes were fixed with an epoxy-based potting agent such that the hollow parts of the hollow fiber membranes were not clogged. Thus, a mini module (inner surface area 53 $cm^2$) was prepared. The mini module was washed with ultrapure water at 37°C for 30 minutes. Subsequently a silicone tube (product name ARAM (registered trademark), inner diameter: 0.8 mm, outer diameter: 1 mm, length: 37 cm) was connected to both ends of the mini module through silicone tubes (product name ARAM (registered trademark), inner diameter: 7 mm, outer diameter: 10 mm, length: 2 cm) and irregular shaped connectors. The above blood plasma (1.5 mL) was perfused in the hollow fiber membranes under a nitrogen atmosphere at 25°C for 4 hours with a flow rate of 0.5 mL/min. The volume of blood plasma per 1 $m^2$ of the surface area of hollow fiber membranes was $2.8 \times 10^2$ mL/$m^2$. In addition, the same perfusion procedure was performed for the silicone tubes alone without using the mini module. The concentration of oxidized LDL in the blood plasma was quantitatively determined before and after the perfusion procedure. The adsorptive removal ratio was calculated by the following formulae.

Adsorptive removal ratio of oxidized LDL (%) =
adsorptive removal ratio of oxidized LDL (%) in mini module
- adsorptive removal ratio of oxidized LDL (%) in silicone tubes alone

Adsorptive removal ratio of oxidized LDL (%) = 100 × (concentration before perfusing - concentration after perfusing) / concentration before perfusing

(EXAMPLE 1)

The above polysulfone film 1 was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.1 weight percent) and ethanol (0.5 weight percent) and was irradiated with γ-ray. The absorbed dose of the γ-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, a polysulfone film having a low soluble hydrophilic polymer ratio, high hydrophilicity, small numbers of adhered blood platelets, and high hematologic compatibility was provided.

(COMPARATIVE EXAMPLE 1)

The above polysulfone film 1 was irradiated with γ-ray in purified water. The absorbed dose of the γ-ray was 28 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the numbers of adhered blood platelets of this film were larger than those of the film in Example 1. Thus, a polysulfone film having low hematologic compatibility was provided.

(COMPARATIVE EXAMPLE 2)

Polyvinylpyrrolidone (K90 from BASF) was used as a hydrophilic polymer and ethanol was used as an antioxidant. The polysulfone film 1 was immersed in an aqueous solution containing the polyvinylpyrrolidone (0.1 weight percent) and ethanol (0.5 weight percent) and was left to stand for three days at room temperature. Subsequently, the film was rinsed with purified water. The film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the contact angle and the numbers of adhered blood platelets of this film were larger than those of the film in Example 1- Thus, a polysulfone film having low hydrophilicity and low hematologic compatibility was provided.

(COMPARATIVE EXAMPLE 3)

The polysulfone film 1 without irradiating with γ-ray was rinsed with purified water. The film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the contact angle and the numbers of adhered blood platelets of this film were larger than those of the film in Example 1. Thus, a polysulfone film having low hydrophilicity and low hematologic compatibility was provided.

Table 1-1

| | Absorbed dose of γ-ray | Hydrophilic polymer | Antioxidant | Surface polyvinylpyrrolidone ratio |
|---|---|---|---|---|
| Example 1 | 27 kGy | Polyvinylpyrrolidone 0.1 wt% | Ethanol 0.5 wt% | 21 wt% |
| Comparative Example 1 | 28 kGy | None | None | < 2 wt% |
| Comparative Example 2 | 0 kGy | Polyvinylpyrrolidone 0.1 wt% | Ethanol 0.5 wt% | 5 wt% |
| Comparative Example 3 | 0 kGy | None | None | < 2 wt% |

Table 1-2

| | Contact angle | Number of adhered human blood platelets (number/ $4.3\times10^3\mu m^2$) | Number of adhered rabbit blood platelets (number/ $10^3\mu m^2$) | Soluble hydrophilic polymer ratio |
|---|---|---|---|---|
| Example 1 | 41° | 0.1 | 0.1 | 0.2 |
| Comparative Example 1 | 43° | 83 | 60 | 0 |
| Comparative Example 2 | 80° | 78 | 50 | 0.1 |
| Comparative Example 3 | 82° | 77 | 58 | 0 |

(EXAMPLE 2)

Polyvinylpyrrolidone (K90 from BASF) was used as a hydrophilic polymer and ethanol was used as an antioxidant. An aqueous solution containing the polyvinylpyrrolidone (0.1 weight percent) and ethanol (0.5 weight percent) was prepared. One thousand milliliters of the aqueous solution was introduced in the blood side and a further 1,000 mL was introduced in the dialysate side of the above hollow fiber membrane module 1 so that the module was filled with the aqueous solution. Subsequently, the module was irradiated with γ-ray. The absorbed dose of the γ-ray was 29 kGy. The dissolution test of polyvinylpyrrolidone was performed using this module. As a result, the amount of dissolution of polyvinylpyrrolidone was 0.15 mg/$m^2$. A hollow fiber in the module was cut into pieces to evaluate the surface polyvinylpyrrolidone ratio, the soluble hydrophilic polymer ratio, and the numbers of adhered blood platelets. Table 2 shows the results.

(EXAMPLE 3)

Polyvinylpyrrolidone (K90 from BASF) was used as a hydrophilic polymer and sodium pyrosulfite was used as an antioxidant. An aqueous solution containing the polyvinylpyrrolidone (0.1 weight percent) and sodium pyrosulfite (500 ppm) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. Table 2 shows the results.

(COMPARATIVE EXAMPLE 4)

One thousand milliliters of purified water was introduced in the blood side and a further 1,000 mL was introduced in the dialysate side of the hollow fiber membrane module 1 so that the module was filled with the purified water. Subsequently, the module was irradiated with γ-ray. The absorbed dose of the γ-ray was 28 kGy. A hollow fiber in the module was cut into pieces to evaluate the surface polyvinylpyrrolidone ratio, the soluble hydrophilic polymer ratio, and

the numbers of adhered blood platelets. As a result, as shown in Table 2, the numbers of adhered blood platelets of this membrane were larger than those of the membranes in Examples 2 and 3. In the filling fluid in the blood side of the module, the maximum increasing value of ultraviolet absorption value in the wavelength range of 260 to 300 nm, the increase being caused by irradiating with γ-ray, was also measured. Furthermore, a mini module was prepared using the same hollow fiber membranes as used in the hollow fiber membrane module 1. The mini module was used for the adsorption test of the oxidized LDL. As shown in Table 3, the adsorptive removal ratio of oxidized LDL of this membrane was lower than that of a hollow fiber membrane on which a cationic hydrophilic polymer was immobilized.

(COMPARATIVE EXAMPLE 5)

Polyvinylpyrrolidone (K90 from BASF) was used as a hydrophilic polymer. An aqueous solution containing the polyvinylpyrrolidone (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3.

(COMPARATIVE EXAMPLE 6)

Ethanol was used as an antioxidant. An aqueous solution containing ethanol (0.5 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3.

(COMPARATIVE EXAMPLE 7)

Sodium pyrosulfite was used as an antioxidant. An aqueous solution containing sodium pyrosulfite (500 ppm) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3.

(COMPARATIVE EXAMPLE 8)

Polyvinylpyrrolidone (K90 from BASF) was used as a hydrophilic polymer and ethanol was used as an antioxidant. An aqueous solution containing the polyvinylpyrrolidone (0.1 weight percent) and ethanol (0.5 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was left to stand for three days at room temperature. The dissolution test of polyvinylpyrrolidone was performed using this module. As a result, the amount of dissolution of polyvinylpyrrolidone was 0.68 $mg/m^2$, which was larger than that of the membrane in Example 2. A hollow fiber in the module was cut into pieces to evaluate the surface polyvinylpyrrolidone ratio, the soluble hydrophilic polymer ratio, and the numbers of adhered blood platelets. Table 2 shows the results. This membrane was not irradiated with γ-ray. Therefore, the numbers of adhered blood platelets were small. However, the amount of dissolution of polyvinylpyrrolidone was large because a grafting reaction or a crosslinking of the polyvinylpyrrolidone was not performed.

Table 2-1

| | Absorbed dose of γ-ray | Hydrophilic polymer | Antioxidant |
|---|---|---|---|
| Example 2 | 29 kGy | Polyvinylpyrrolidone 0.1 wt% | Ethanol 0.5 wt% |
| Example 3 | 29 kGy | Polyvinylpyrrolidone 0.1 wt% | Sodium pyrosulfite 500ppm |

(continued)

| | Absorbed dose of γ-ray | Hydrophilic polymer | Antioxidant |
|---|---|---|---|
| Comparative Example 4 | 28 kGy | None | None |
| Comparative Example 5 | 29 kGy | Polyvinylpyrrolidone 0.1 wt% | None |
| Comparative Example 6 | 29 kGy | None | Ethanol 0.5 wt% |
| Comparative Example 7 | 29 kGy | None | Sodium pyrosulfite 500ppm |
| Comparative Example 8 | 0 kGy | Polyvinylpyrrolidone 0.1 wt% | Ethanol 0.5 wt% |

Table 2-2

| | Number of adhered human blood platelets (number/$4.3\times10^3\mu m^2$) | Number of adhered rabbit blood platelets (number/$10^3\mu m^3$) | Soluble hydrophilic polymer ratio (%) |
|---|---|---|---|
| Example 2 | 0.1 | 0.1 | 9 |
| Example 3 | 0.1 | 0.1 | 8.5 |
| Comparative Example 4 | 65 | 48 | 3.5 |
| Comparative Example 5 | 30 | 25 | 3.6 |
| Comparative Example 6 | 25 | 22 | 9.7 |
| Comparative Example 7 | 31 | 18 | 9.5 |
| Comparative Example 8 | 0.5 | 1 | 73.3 |

(EXAMPLE 4) Reference

Polyvinylpyrrolidone (K90 from BASF) was used as a nonionic hydrophilic polymer and polyethyleneimine (weight-average molecular weight: 1,000,000, from BASF) was used as a cationic hydrophilic polymer. An aqueous solution containing the polyvinylpyrrolidone (0.1 weight percent) and the polyethyleneimine (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 27 kGy. In the filling fluid in the blood side of the module, the maximum increasing value of ultraviolet absorption value in the wavelength range of 260 to 300 nm, the increase being caused by irradiating with γ-ray, was measured. A hollow fiber in the module was cut into pieces to evaluate the number of adhered blood platelets. Furthermore, a mini module was prepared using the same hollow fiber membranes as used in the hollow fiber membrane module 1. The mini module was used for the adsorption test of the oxidized LDL. Results shown in Table 3 were obtained. Table 3 shows the results.

(EXAMPLE 5) Reference

Polyethyleneimine (weight-average molecular weight: 1,000,000, from BASF) was used as a cationic hydrophilic polymer and ethanol was used as an antioxidant. An aqueous solution containing the polyethyleneimine (0.1 weight percent) and ethanol was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. In the filling fluid in the blood side of the module, the maximum increasing value of ultraviolet absorption value in the wavelength range of 260 to 300 nm, the increase being caused by irradiating with γ-ray, was measured. As a result, as shown in Table 3, the maximum increasing value of ultraviolet absorption value of this membrane is lower than that of the membrane in Comparative Example 9. A hollow fiber in the module was cut into pieces to evaluate the

number of adhered blood platelets. Furthermore, a mini module was prepared using the same hollow fiber membranes as used in the hollow fiber membrane module 1. The mini module was used for the adsorption test of the oxidized LDL. Table 3 shows the results.

(COMPARATIVE EXAMPLE 9)

Polyethyleneimine (weight-average molecular weight: 1,000,000, from BASF) was used as a cationic hydrophilic polymer. An aqueous solution containing the polyethyleneimine (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 28 kGy. In the filling fluid in the blood side of the module, the maximum increasing value of ultraviolet absorption value in the wavelength range of 260 to 300 nm, the increase being caused by irradiating with γ-ray, was measured. A hollow fiber in the module was cut into pieces to evaluate the number of adhered blood platelets. Furthermore, a mini module was prepared using the same hollow fiber membranes as used in the hollow fiber membrane module 1. The mini module was used for the adsorption test of the oxidized LDL. As a result, as shown in Table 3, the number of adhered blood platelets of this membrane was larger than that of the membrane in Example 4.

Table 3-1

| | Absorbed dose of γ-ray | Nonionic hydrophilic polymer | Cationic hydrophilic polymer | Antioxidant |
|---|---|---|---|---|
| Example 4* | 27 kGy | Polyvinylpyrrolidone 0.1 wt% | Polyethyleneimine 0.1 wt% | None |
| Example 5* | 29 kGy | None | Polyethyleneimine 0.1 wt% | Ethanol 0.5 wt% |
| Comparative Example 9 | 28 kGy | None | Polyethyleneimine 0.1 wt% | None |
| Comparative Example 4 | 28 kGy | None | None | None |

Table 3-2

| | Number of adhered human blood platelets (number/ $4.3\times10^3 \mu m^2$) | Soluble hydrophilic polymer ratio (%) | Maximum increasing value of ultraviolet absorption value | Adsorptive removal ratio of oxidized LDL (%) |
|---|---|---|---|---|
| Example 4* | 0.2 | 10 | 0.60 | 26 |
| Example 5* | 12 | 12 | 0.25 | 27 |
| Comparative Example 9 | 14 | 8.7 | 0.61 | 30 |
| Comparative Example 4 | 65 | 3.5 | 0.15 | 10 |
| *Reference Example | | | | |

(EXAMPLE 6)

Five thousand milliliters of ultrapure water at 40°C was introduced in the blood side and a further 5,000 mL of ultrapure water at 40°C was introduced in the dialysate side of the above hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.075 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module.

Table 4 shows the results.

(EXAMPLE 7) Reference

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(EXAMPLE 8) Reference

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyvinylpyrrolidone (K90 from ISP) was used as a hydrophilic polymer. An aqueous solution containing the polyvinylpyrrolidone (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The adhering test of blood platelets and the adsorption test of IL-6 were performed with the module. Table 4 shows the results.

(COMPARATIVE EXAMPLE 10)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.010 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 11)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (polyethylene glycol #200 from Nacalai Tesque, Inc.) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 12)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Mw 900,000 from Scientific Polymers Products, Inc.) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 13)

Five thousand milliliters of ultrapure water at 40°C was introduced in the blood side and a further 5,000 mL of ultrapure water at 40°C was introduced in the dialysate side of the hollow fiber membrane module 2 in order to wash the module. Subsequently, the module was filled with ultrapure water and was irradiated with γ-ray. The absorbed dose of the γ-ray was 28 kGy. The adhering test of blood platelets and the adsorption test of IL-6 were performed with the module. Table 4 shows the results.

Table 4

| | Number of adhered human blood platelets (number/$4.3\times10^3\mu m^2$) | Adsorptivity to IL-6 ($ng/cm^2$) | Immobilization density of polyethylene glycol ($mg/m^2$) |
|---|---|---|---|
| Example 6* | 0.56 | 0.209 | 384 |
| Example 7* | 0.43 | 0.180 | 353 |
| Example 8* | 0.99 | 0.163 | - |
| Comparative Example 10 | 3.23 | 0.032 | 137 |
| Comparative Example 11 | 48.59 | 0.282 | 172 |
| Comparative Example 12 | 0.56 | 0.053 | 239 |
| Comparative Example 13 | 100 or more | 0.162 | 0 |
| *Reference Examples | | | |

(EXAMPLE 9)

A connector part at the blood side of an artificial kidney module of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 10)

A blood tube part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 11)

A blood chamber part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 12)

A mesh part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with $\gamma$-ray. The adhering test of blood platelets was performed. Table 5 shows the result.

(COMPARATIVE EXAMPLE 14)

A connector part at the blood side of an artificial kidney module of a commercial blood circuit for an artificial kidney (artificial kidney blood circuit H-102-KTS from Toray Medical Co., Ltd) was cut into small pieces to perform the adhering test of blood platelets. Table 5 shows the result.

(COMPARATIVE EXAMPLE 15)

A blood tube part of a commercial blood circuit for an artificial kidney (artificial kidney blood circuit H-102-KTS from Toray Medical Co., Ltd) was cut into small pieces to perform the adhering test of blood platelets. Table 5 shows the result.

(COMPARATIVE EXAMPLE 16)

A blood chamber part of a commercial blood circuit for an artificial kidney (artificial kidney blood circuit H-102-KTS from Toray Medical Co., Ltd) was cut into small pieces to perform the adhering test of blood platelets. As a result, as shown in Table 5, the number of adhered blood platelets was 7.0 ($/4.3\times10^3\ \mu m^2$).

(COMPARATIVE EXAMPLE 17)

A mesh part of a commercial blood circuit for an artificial kidney (artificial kidney blood circuit H-102-KTS from Toray Medical Co., Ltd) was cut into small pieces to perform the adhering test of blood platelets. Table 5 shows the result.

Table 5

| | Number of adhered human blood platelets(number/$4\text{-}3\times10^3\mu m^2$) |
|---|---|
| Example 9 | 0.67 |
| Example 10 | 0.67 |
| Example 11 Example 12 | 0.33 |
| | 29.00 |
| Comparative Example 14 | 5.67 |
| Comparative Example 15 | 3.33 |
| Comparative Example 16 | 7.00 |
| Comparative Example 17 | 100 or more |

(EXAMPLE 13) Reference

A commercial glassy carbon plate (from Toyo Tanso Co., Ltd.) was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.01 weight percent) and ethanol (0.1 weight percent) and was irradiated with $\gamma$-ray. The absorbed dose of the $\gamma$-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was measured. The contact angle of the film was 39

degrees, whereas that of an untreated film was 98 degrees. This result showed that the film was significantly subjected to hydrophilization.

(EXAMPLE 14) Reference

A commercial glassy carbon plate (from Toyo Tanso Co., Ltd.) was used as a substrate. Polyvinylpyrrolidone (K90 from BASF) was used as a hydrophilic polymer. The substrate was immersed in an aqueous solution containing the polyvinylpyrrolidone (0.01 weight percent) and was irradiated with γ-ray. The absorbed dose of the γ-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was.measured. The contact angle of the film was 52 degrees, whereas that of the untreated film was 98 degrees. This result showed that the film was significantly subjected to hydrophilization.

(COMPARATIVE EXAMPLE 18)

The glassy carbon plate used in Example 13 was irradiated with γ-ray in purified water. The absorbed dose of the γ-ray was 28 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The contact angle of the surface of the film was 98 degrees, which was the same as the 98 degrees of the untreated film.

(EXAMPLE 15) Reference

A commercial carbon sheet (from Toray Industries, Inc.) was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.1 weight percent) and ethanol (0.1 weight percent) and was irradiated with γ-ray. The absorbed dose of the γ-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was measured. The contact angle of the film was 30 degrees, whereas that of an untreated film was 131 degrees. This result showed that the film was significantly subjected to hydrophilization.

Industrial Applicability

According to a modified substrate prepared according to the method of the present invention, a hydrophilic polymer is immobilized on the surface, and in addition, excessive crosslinking, degradation or the like of the hydrophilic polymer is prevented. Accordingly, the adhesion of organic matter such as proteins, or biogenic substances can be suppressed. In particular, the modified substrate has high hematologic compatibility. Furthermore, the high hematologic compatibility can be achieved while the adsorption of a cytokine is maintained.

The modified substrate can be widely used for applications that require hydrophilicity on the surface. For example, the modified substrate can be preferably used in medical devices such as an artificial blood vessel, a catheter, a blood bag, a blood filter, a contact lens, an intraocular lens, an artificial kidney, an artificial lung, and auxiliary instruments for surgical operation. The modified substrate can be preferably used in separation membranes of biogenic substances such as amino acids, peptides, saccharides, proteins, and composites thereof. The modified substrate can be preferably used in instruments used for biological experiments such as pipette tips, tubes, Petri dishes, and sample collection tubes; bioreactors; molecular motors; DDS; protein chips; DNA chips; biosensors; and components of analytical instruments such as an atomic force microscope (AFM), a scanning near-field optical microscope (SNOM), and a surface plasmon resonance (SPR) sensor. In addition, the modified substrate can be preferably used in separation membranes for water treatment such as membranes for a water purifier, membranes for purifying clean water, membranes for purifying sewage, and reverse osmosis (RO) membranes. In particular, the modified substrate is preferably used for applications in which the substrate is brought into contact with a biogenic substance, for example, a module for blood purification such as an artificial kidney.

## Claims

1. A method for producing a modified substrate comprising a step of irradiating the substrate with γ-ray radiation with an absorbed dose of at least 20 kGy while the substrate is brought into contact with an aqueous solution containing a hydrophilic polymer and an antioxidant, wherein the hydrophilic polymer is a polyvinylpyrrolidone or a polyvinylpyrrolidone copolymer, and the substrate is a separation membrane system comprising polysulfone and polyvinylpyrrolidone,
wherein the antioxidant is selected from alcohols,
and inorganic salts, wherein the alcohols are selected from methanol, ethanol, propanol, ethylene glycol and glycerine, and the inorganic salts are selected from sodium hydrosulfite, sodium pyrosulfite, and sodium dithionate,
and wherein the obtained modified substrate has a soluble hydrophilic polymer ratio of 15 weight percent or less, a number of adhered human blood platelets adhered in an area of $4.3x10^3$ $\mu m^2$ of 10 or less, and a surface hydrophilic polymer ratio measured by X-ray photoelectron spectrometry of at least 20 weight percent, wherein the soluble hydrophilic polymer ratio is defined as the ratio of soluble hydrophilic polymer which is soluble in a solvent that dissolves the hydrophilic polymer but does not dissolve the substrate to the total of the hydrophilic polymer in the modified substrate, and wherein the number of adhered blood platelets is defined as the number of blood platelets adhered on the surface of the modified substrate when it is brought into contact with blood for one hour, where the soluble hydrophilic polymer ratio, the number of adhered human blood platelets and the surface hydrophilic polymer ratio are measured as defined in the description.

2. The method for producing a modified substrate according to claim 1, wherein the substrate is immersed in the aqueous solution containing a hydrophilic polymer and an antioxidant in order to bring the substrate into contact with the aqueous solution.

3. The method for producing a modified substrate according to claim 1, wherein the separation membrane is a hollow fiber membrane.

4. The method for producing a modified substrate according to claim 3, wherein the inside of the hollow fiber membrane is filled with the aqueous solution containing a hydrophilic polymer and an antioxidant in order to bring the hollow fiber membrane into contact with the aqueous solution.

5. The method for producing a modified substrate according to claim 4, wherein the outside of the hollow fiber membrane is further brought into contact with the aqueous solution.

6. The method for producing a modified substrate according to claim 1, wherein the aqueous solution is filtered through the separation membrane in order to bring the separation membrane into contact with the aqueous solution.

7. A method for producing a modified substrate according to claim 1, comprising a step of irradiating a plurality of substrates which are a port element, a separation membrane and a circuit with γ-ray radiation at the same time while the substrates are brought into contact with an aqueous solution containing a hydrophilic polymer and an antioxidant.

## Patentansprüche

1. Verfahren zur Herstellung eines modifizierten Substrats, das einen Schritt zur Bestrahlung des Substrats mit γ-Strahlen-Bestrahlung mit einer absorbierten Dosis von zumindest 20 kGy umfasst, während das Substrat mit einer wässrigen Lösung in Kontakt gebracht wird, die ein hydrophiles Polymer und ein Antioxidans umfasst, wobei das hydrophile Polymer ein Polyvinylpyrrolidon oder ein Polyvinylpyrrolidon-Copolymer ist und das Substrat ein Membrantrennsystem ist, das Polysulfon und Polyvinylpyrrolidon umfasst,
wobei das Antioxidans aus Alkoholen und anorganischen Salzen ausgewählt ist, wobei die Alkohole aus Methanol, Ethanol, Propanol, Ethylenglykol und Glycerin ausgewählt sind und die anorganischen Salze aus Natriumhydrogensulfit, Natriumpyrosulfit und Natriumdithionat ausgewählt sind,
und wobei das erhaltene modifizierte Substrat einen Anteil an löslichem hydrophilem Polymer von 15 Gew.-% oder weniger, eine Anzahl an anhaftenden menschlichen Blutplättchen, die auf einer Fläche von 4,3 x $10^3$ $\mu m^2$ anhaften, von 10 oder weniger und einen Anteil an hydrophilem Oberflächenpolymer, gemessen durch Röntgenphotoelektronenspektrometrie von zumindest 20 Gew.-% aufweist, wobei der Anteil des löslichen hydrophilen Polymers als Anteil des löslichen hydrophilen Polymers, der in einem Lösungsmittel löslich ist, welches das hydrophile Polymer

löst, aber das Substrat nicht löst, an der Gesamtmenge des hydrophilen Polymers im modifizierten Substrat definiert ist und wobei die Anzahl an anhaftenden Blutplättchen als Anzahl der auf der Oberfläche des modifizierten Substrats anhaftenden Blutplättchen, wenn es eine Stunde lang mit Blut in Kontakt kommt, definiert ist, wobei der Anteil des löslichen hydrophilen Polymers, die Anzahl an anhaftenden Blutplättchen und der Anteil an hydrophilem Oberflächenpolymer wie in der Beschreibung definiert gemessen werden.

**2.** Verfahren zur Herstellung eines modifizierten Substrats nach Anspruch 1, wobei das Substrat in eine wässrigen Lösung getaucht wird, die ein hydrophiles Polymer und ein Antioxidans umfasst, um das Substrat mit einer wässrigen Lösung in Kontakt zu bringen.

**3.** Verfahren zur Herstellung eines modifizierten Substrats nach Anspruch 1, wobei die Trennmembran eine Hohlfasermembran ist.

**4.** Verfahren zur Herstellung eines modifizierten Substrats nach Anspruch 3, wobei der Innenraum der Hohlfasermembran mit einer wässrigen Lösung gefüllt ist, die ein hydrophiles Polymer und ein Antioxidans umfasst, um die Hohlfasermembran mit der wässrigen Lösung in Kontakt zu bringen.

**5.** Verfahren zur Herstellung eines modifizierten Substrats nach Anspruch 4, wobei die Außenseite der Hohlfasermembran weiters mit einer wässrigen Lösung in Kontakt gebracht wird.

**6.** Verfahren zur Herstellung eines modifizierten Substrats nach Anspruch 1, wobei die wässrige Lösung durch die Trennmembran filtriert wird, um die Trennmembran mit der wässrigen Lösung in Kontakt zu bringen.

**7.** Verfahren zur Herstellung eines modifizierten Substrats nach Anspruch 1, das einen Schritt der Bestrahlung einer Vielzahl von Substraten, die ein Anschlusselement, eine Trennmembran und ein Schaltkreis umfassen, mittels γ-Strahlen-Bestrahlung zur gleichen Zeit, während die Substrate mit einer wässrigen Lösung, die ein hydrophiles Polymer und ein Antioxidans umfasst, in Kontakt gebracht werden, umfasst.

## Revendications

**1.** Procédé de production d'un substrat modifié comprenant une étape d'irradiation du substrat par un rayonnement γ avec une dose absorbée d'au moins 20 kGy tandis que le substrat est amené en contact avec une solution aqueuse contenant un polymère hydrophile et un antioxydant,
dans lequel le polymère hydrophile est une polyvinylpyrrolidone ou un copolymère de polyvinylpyrrolidone, et le substrat est un système à membrane de séparation comprenant de la polysulfone et de la polyvinylpyrrolidone, dans lequel l'antioxydant est choisi parmi les alcools et les sels inorganiques, dans lequel les alcools sont choisis parmi le méthanol, l'éthanol, le propanol, l'éthylène glycol et la glycérine, et les sels inorganiques sont choisis parmi l'hydrosulfite de sodium, le pyrosulfite de sodium et le dithionate de sodium,
et dans lequel le substrat modifié obtenu a un rapport de polymère hydrophile soluble de 15 pour cent en poids ou moins, un nombre de plaquettes sanguines humaines collées étant collées dans une zone de 4,3 x $10^3$ $\mu m^2$ de 10 ou moins, et un rapport de polymère hydrophile de surface mesuré par spectrométrie photoélectronique à rayons X d'au moins 20 pour cent en poids, dans lequel le rapport de polymère hydrophile soluble est défini comme étant le rapport de polymère hydrophile soluble qui est soluble dans un solvant qui dissout le polymère hydrophile mais qui ne dissout pas le substrat au total du polymère hydrophile dans le substrat modifié, et dans lequel le nombre de plaquettes sanguines collées est défini comme étant le nombre de plaquettes sanguines étant collées sur la surface du substrat modifié lorsqu'il est amené en contact avec du sang pendant une heure, où le rapport de polymère hydrophile soluble, le nombre de plaquettes sanguines humaines collées et le rapport de polymère hydrophile de surface sont mesurés tel que défini dans la description.

**2.** Procédé de production d'un substrat modifié selon la revendication 1, dans lequel le substrat est immergé dans la solution aqueuse contenant un polymère hydrophile et un antioxydant afin d'amener le substrat en contact avec la solution aqueuse.

**3.** Procédé de production d'un substrat modifié selon la revendication 1, dans lequel la membrane de séparation est une membrane à fibres creuses.

**4.** Procédé de production d'un substrat modifié selon la revendication 3, dans lequel l'intérieur de la membrane à fibres

creuses est rempli de la solution aqueuse contenant un polymère hydrophile et un antioxydant afin d'amener la membrane à fibres creuses en contact avec la solution aqueuse.

**5.** Procédé de production d'un substrat modifié selon la revendication 4, dans lequel l'extérieur de la membrane à fibres creuses est en outre amené en contact avec la solution aqueuse.

**6.** Procédé de production d'un substrat modifié selon la revendication 1, dans lequel la solution aqueuse est filtrée à travers la membrane de séparation afin d'amener la membrane de séparation en contact avec la solution aqueuse.

**7.** Procédé de production d'un substrat modifié selon la revendication 1, comprenant une étape d'irradiation d'une pluralité de substrats qui sont un élément d'orifice, une membrane de séparation et un circuit par un rayonnement $\gamma$ au même moment tandis que les substrats sont amenés en contact avec une solution aqueuse contenant un polymère hydrophile et un antioxydant.

Fig. 1

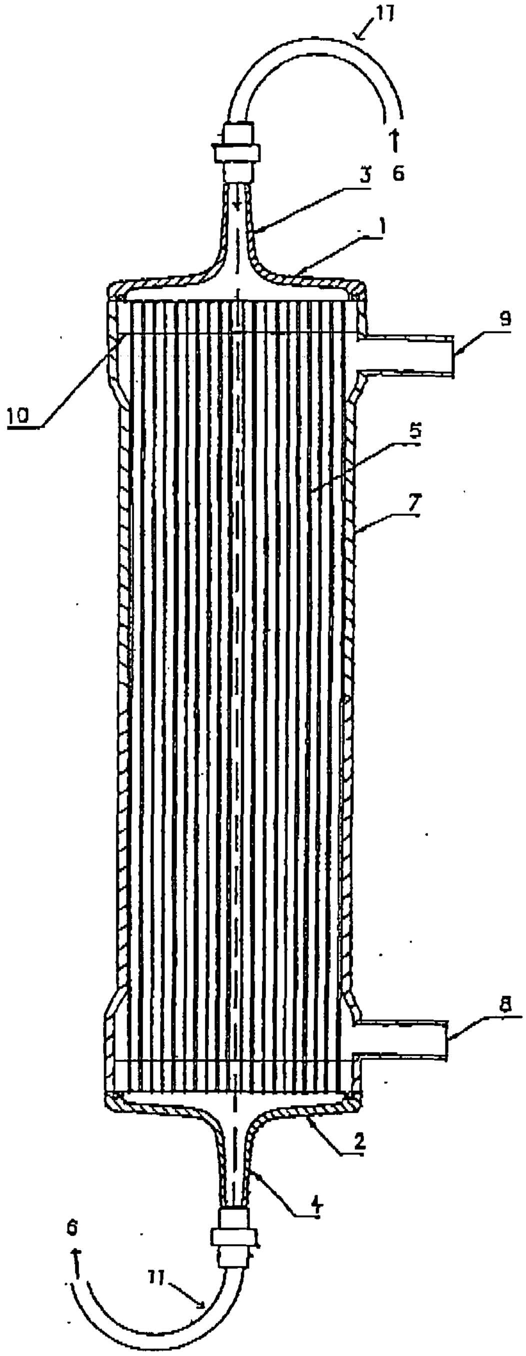

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 10118472 A
- JP 6238139 A
- US 5240747 A
- EP 0472936 A
- WO 02062880 A
- JP 63097634 A
- US 5658466 A
- JP 2000237557 A

### Non-patent literature cited in the description

- **H. ITABE et al.** *J. Biol. Chem.,* 1994, vol. 269, 15274